# EUROPÄISCHE PATENTSCHRIFT

(11) **EP 2 163 612 B1**
(45) Veröffentlichungstag und Bekanntmachung des Hinweises auf die Patenterteilung: **30.11.2016**
(21) Anmeldenummer: 08016161.5
(22) Anmeldetag: 13.09.2008
(51) Int. Cl.: C12M 1/04, C12M 1/00, C12M 1/02

(54) **Kultiviereinheit**
Cultivation device
Unité de culture

(43) Veröffentlichungstag der Anmeldung: 17.03.2010
(73) Patentinhaber: Airbus DS GmbH, 82024 Taufkirchen (DE)
(72) Erfinder: Kern, Peter, Dr., 88682 Salem (DE); Brendle, Klaus, 78247 Hilzingen (DE)
(74) Vertreter: Daub, Thomas

(56) Entgegenhaltungen:
- EP-A- 0 889 119
- EP-A- 1 571 201
- WO-A-2007/083465
- US-A- 4 783 413

## Beschreibung

Die Erfindung betrifft eine Kultiviereinheit, insbesondere zur Kultivierung von adherenten und nicht adherenten Zellsubstraten, aquatischer Kleinstlebewesen oder Keimen pflanzlichen unter variablen Schwerkraftbedingungen (Hyper-g, d.h. kleiner 1 g bis hin zu Schwerelosigkeit, d.h. µg, im Weltraum) im Rahmen wissenschaftlicher Experimente.

Bei derartigen Experimenten ist insbesondere eine zuverlässige, kontrollierte und vollautomatische Funktion der Kultiviereinheit von wesentlicher Bedeutung. Dies betrifft insbesondere:
- den vollständigen Fluidwechsel durch mehrfaches, vollständiges Austauschen der Flüssigkeit im Experimentiervolumen durch eine andere Flüssigkeit sowie die
- Probenentnahme und Probenspeicherung.

Aus dem US-Patent US 4,783,413 A ist eine Vorrichtung zur Versorgung einer Kultivierkammer mit einem Fluid aus einer Versorgungskammer bekannt, wobei das Fluid mittels einer in der Versorgungskammer angeordneten osmotischen Pumpe in die Kultivierkammer gefördert wird.

Aufgabe der vorliegenden Erfindung ist es, eine Kultiviereinheit zu schaffen, welche diesen Anforderungen genügt.

Diese Aufgabe wird mit dem Gegenstand des Patentanspruchs 1 gelöst. Vorteilhafte Ausführungen sind Gegenstand von Unteransprüchen.

Die Erfindung wird anhand von konkreten Ausführungsbeispielen unter Bezugnahme auf Fig. näher erläutert. Es zeigen:
- Fig. 1: eine erfindungsgemäße Kultiviereinheit;
- Fig. 2: die Versorgungseinheit einer erfindungsgemäßen Kultiviereinheit im gesicherten Zustand (schematische Darstellung);
- Fig. 3: die Versorgungseinheit nach Fig. 2 in aktiviertem Zustand (schematische Darstellung);
- Fig. 4: die Versorgungseinheit einer erfindungsgemäßen Kultiviereinheit im gesicherten Zustand;
- Fig. 5: die Versorgungseinheit einer erfindungsgemäßen Kultiviereinheit im aktivierten Zustand mit entleerten Fluidbehälter.

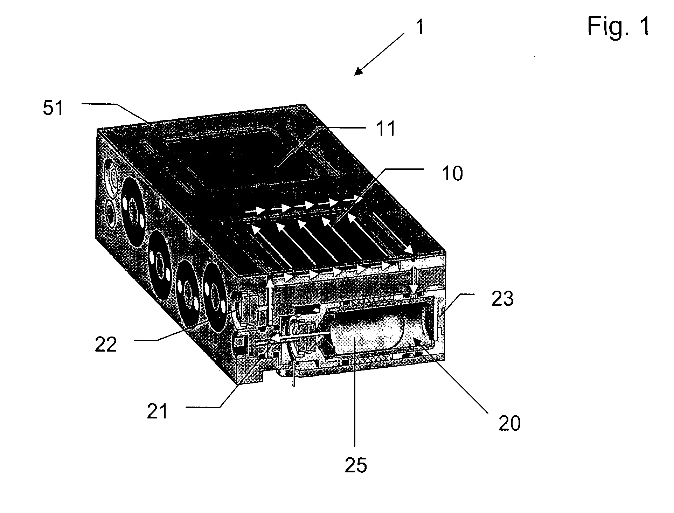

Generell bezeichnen gleiche Bezugsziffern in Fig. 1 bis 5 denselben Gegenstand.

Fig. 1 zeigt eine Kultiviereinheit 1 gemäß der Erfindung in einer vertikalen Schnittdarstellung. Sie dient zur Kultivierung von adherenten und nicht adherenten Zellsubstraten, aquatischer Kleinstlebewesen oder Keimen pflanzlichen Ursprunges für die experimentelle Erforschung unter variablen Schwerkraftbedingungen. Die Funktionselemente dieser Einheit können auch zur automatisierten Durchführung von mehrstufigenstufigen Probenbehandlungsschritten verwendet werden.

Die Kultiviereinheit 1 stellt ein modulares System dar, bestehend aus einer oder mehreren Kultivierkammern (in Fig. 1 sind zwei Kultivierkammern 10,11 zu erkennen), wobei jeder Kultivierkammer 10,11 mehrere, z.B. bis zu acht, mit unterschiedlichen Fluiden 25 befüllte Versorgungseinheiten 20 zugeordnet werden können. In der in Fig. 1 gezeigten konkreten Ausführung umfasst die Kultiviereinheit 1 drei, parallel zueinander angeordnete Versorgungseinheiten (davon ist nur eine, 20, direkt zu erkennen), die alle zur Versorgung derselben Kultivierkammer 10 vorgesehen sind. Bei den in den Vorsorgungseinheiten 20 gespeicherten Fluiden 25 kann es sich z.B. um Zellsubstrate, Aktivatoren, Fixative, neutrale Lösungen, Reagenzien, Antikörperlösungen, etc handeln.

Die Deckfläche einer Kultivierkammer kann wahlweise (entsprechend dem Sauerstoffbedarf / Gasproduktion der Zell- /Pflanzeneinheiten) durch eine gaspermeable Folie oder durch eine gasdichte verschraubte Dichtfläche, mit optischen klaren oder nichttransparenten Eigenschaften, erfolgen. In der in Fig. 1 gezeigten Ausführung sind die Kultivierkammern 10,11 durch eine gaspermeable, transparente Folie 51 abgedeckt, die auf die obere Fläche der Kultiviereinheit 1 aufgeklebt ist.

Da sich adherente Zellen unter µg-Einfluss an sämtlichen Berandungen der Kultivierkammer ansiedeln, kann eine austauschbare Oberfläche in Form eines Inserts / Folie etc. vorgesehen werden, das nach Abschluss des Experimentes aus der Kultivierkammer entnommen und analytisch (durch mikroskopische Auswertung adherenter Zellanhaftungen) und chemisch (DNA) bewertet werden kann.

Die Strömungspfade des Fluids 25 sind in Fig. 1 ebenfalls eingezeichnet. Wie weiter unten noch im Detail dargelegt wird, verlässt das in der Versorgungseinheit 20 gespeicherte Fluid 25 die Vorsorgungseinheit 20 über ein Septen-Nadel-Interface 21 und wird in die Kultivierkammer 10 geleitet. Dort verdrängt es die gesamt oder einen Teil des dort vorhandenen Mediums, welches in den rückwärtigen Teil 23 der Versorgungseinheit 20 geleitet wird. Die Austauschmenge wird dort gespeichert und kann zur weiteren Analyse daraus entnommen werden. Die Befüllung der Versorgungseinheit 20 erfolgt über den Anschluss 22. An der Einrichtung 29 können Einstellungen hinsichtlich Zeitpunkt, Menge, und Strömungswiderstand des Strömungsvorgangs vorgenommen werden.

Durch die Kombination unterschiedlicher Kammergeometrien mit einer variablen Anzahl von aufgeladenen Flüssigkeitsspeichern ergibt sich eine Vielzahl möglicher Anwendungen für wissenschaftliche Experimente im Bereich von Hyper-g bis hin zu µg-Bedingungen. Die hierbei verwendeten Versorgungsmodule 20 sind in Ihrer Größe skalierbar. Die Menge und Konzentration der eingebrachten Lösung ist ebenfalls variabel und hängt von der verwendeten Baugröße (Durchmesser/Länge der Versorgungseinheit) und dem Bedarf des Experimentes ab.

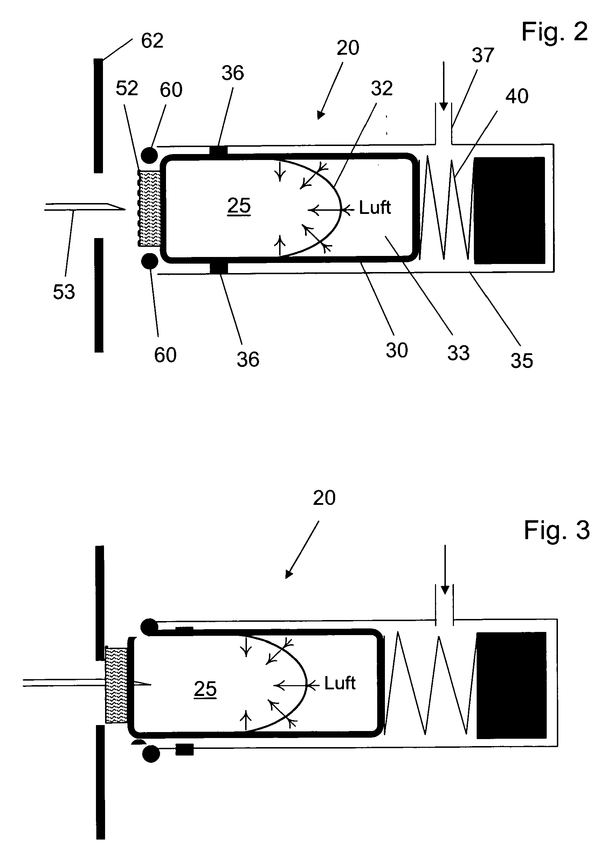

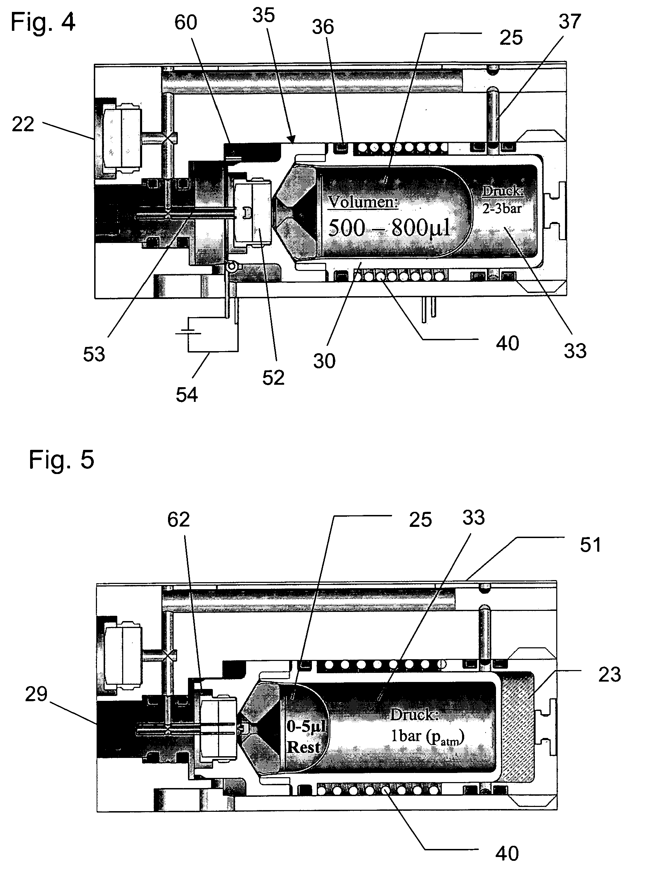

Fig. 2 und 4 zeigen (Fig. 2 in schematischer, Fig. 4 in realistischer Darstellung) die Versorgungseinheit 20 einer erfindungsgemäßen Kultiviereinheit 1 im gesicherten Zustand. Gesicherter Zustand bedeutet dabei, dass das Fluid 25 sich innerhalb eines in seiner Lage fixierten Fluidbehälters 30 befindet, der mittels Septum 52 abgeschlossenen ist. Das Innere des Fluidbehälters 30 ist durch eine elastische Membran 32 zweigeteilt in zwei Teilräume, wobei sich in einem Teilraum das Fluid 25 befindet. Der zweite Teilraum 33 kann als gasgefüllte Druckkammer ausgebildet sein, in der ein definierter Druck eingestellt wird, um später das Austreiben des Fluids 25 aus dem Fluidbehälter 30 zu unterstützen. Es handelt sich hierbei jedoch um ein fakultatives Merkmal, denn das Austreiben des Fluids 25 kann auch allein durch die elastischen Kräfte der Membran 32 erzielt werden.

Der Fluidbehälter 30 selbst ist innerhalb eines Druckbehälters 35 durch die Kraft eines elastischen Energiespeichers, hier einer vorgespannten Spiralfeder 40, verschiebbar. Die Dichtungen 36 zwischen Druckbehälter 35 und Fluidbehälter 30 sorgen für eine Abdichtung des Innenraums des Druckbehälters 35 gegen die Umgebung. Der Druckbehälter 35 ist über den Eingang 37 außerdem mit dem Abfluss der zugeordneten Kultivierkammer 1 (Fig. 1) verbunden.

In dem in den Fig. 2,4 gezeigten gesicherten Zustand ist der Fluidbehälter 30 mittels einer ringförmigen Schmelzsicherung 60 in der gezeigten Ausgangsposition fixiert. Dabei wird der Fluidbehälter 30 unter der Kraft der Feder 40 an den konusförmigen Ring der Schmelzsicherung 60 gedrückt. Die Öffnung der Schmelzsicherung 60 erfolgt durch definierte elektrische Heizimpulse. Die Schmelzsicherung 60 besteht aus niedrigschmelzenden Materialien (z.B. Kunststoff, Wachs, Metall), so dass ein Öffnen der Schmelzsicherung bei geringen Temperaturen möglich ist (z.B. ab ca. 60°C). Das Öffnen der Schmelzsicherung 60 bei tiefen Temperaturen hat den Vorteil, dass thermische Schäden an der Zellkultur in der Kultivierkammer und/oder an dem im Fluidbehälter 30 gespeicherten Fluid 25 vermieden werden.

Ein Schmelzen der Schmelzsicherung 60 durch thermische Einwirkung mittels elektrischen Heizeinrichtung 54 (Fig. 4) führt zu einer Spreizung des Rings 60 und somit zum Wegfall der Rückhaltefunktion. Unter der Kraft der vorgespannten Feder 40 bewegt sich der Fluidbehälter 30 bis auf einen justierbaren Anschlag 62 und verbleibt in dieser Position (Fig. 3 und 5).

Dabei durchdringt eine Kanüle 53 das Septum 52 des Fluidbehälters 30 und öffnet somit einen Kanal, durch den das Fluid 25 entweicht und durch die elastische Kraft der Membran 32 mit Unterstützung des Überdrucks innerhalb der Druckkammer 33 in die zugeordnete Kultivierkammer 1 verdrängt werden kann.

Während der Bewegung des Fluidbehälters 30 entsteht auf seiner Rückseite innerhalb des Druckbehälters 35 gleichzeitig ein Unterdruck, wodurch sich aufgrund der Saugwirkung in dem entstehenden Zwischenraum 23 zwischen Fluidbehälter 30 und Druckbehälter 35 eine bestimmte Menge (definiert durch den eingestellten Weg des Druckbehälters) an Medium aus der Kultivierkammer 1 ansammelt (Probe), das zur späteren Analyse genutzt werden kann.

Der gesamte Strömungsvorgang dauert so lange an, bis sich das Druckniveau der Druckkammer 33 innerhalb des Fluidbehälters 30 an das Druckniveau der Kultivierkammer 1 angeglichen hat (siehe Fig. 4 und 5). Bei starrer Abdeckung der Kultivierkammer sind zusätzliche Ausgleichsvolumen vorgesehen, um den Ansaugvorgang durch die Bewegung des Fluidbehälters zu ermöglichen. In den Fig. 4 und 5 sind beispielhafte Werte für das Fluidvolumen sowie für den Druck in der Druckkammer vor Beginn und am Ende des Strömungsvorgangs angegeben.

Wie bereits erwähnt, ist die Ausbildung des zweiten Teilraums 33 innerhalb des Fluidbehälterns 30 als Druckkammer nicht zwingend. Falls in diesem zweiten Teilraum 33 auf der dem Fluid abgewandten Seite der elastischen Membran 32 kein Überdruck eingebracht wird, ist es vorteilhaft, diesen Teilraum 33 mit dem Innern des umgebenden Druckbehälters 35 zu verbinden.

Mit der Erfindung werden insgesamt insbesondere die folgenden Vorteile erzielt:
- zuverlässiger und kontrollierter Medienwechsel (bis zu 8x) mit gleichzeitiger Probenentnahme und Probenspeicherung;
- vollständiger Medienwechsel (nicht nur Verdünnung der in der Kultivierkammer vorhandenen Flüssigkeit);
- Beobachtung der Kultivierkammer mit optischer Qualität und optischen Sensoren möglich;
- sequentielle, selektive und vollautomatische Aktivierung der einzelnen Versorgungsmodule durch gezielte Ansteuerung der betreffenden Schmelzsicherungen;
- Auslösen der Schmelzsicherungen bei niedrigen Temperaturen.

## Patentansprüche

1. Kultiviereinheit (1), umfassend eine Kultivierkammer (10) sowie eine oder mehrere hiermit in Fließverbindung stehende Versorgungseinheiten (20) zur Zuführung eines Fluids (25), **dadurch gekennzeichnet, dass** eine Versorgungseinheit (20) folgende Merkmale aufweist:
- einen innerhalb eines Druckbehälters (35) unter dem Einfluss eines elastischen Energiespeichers (40) verschiebbaren Fluidbehälter (30) mit dem darin speicherbaren Fluid (25),
- eine Schmelzsicherung (60) zur Aktivierung der Bewegung des Fluidbehälters (30),
- eine Kanüle (53), die ein am Abfluss des Fluidbehälters (30) vorhandenes Septum (52) durchdringt, wenn die Bewegung des Fluidbehälters (30) unter dem Einfluss des elastischen Energiespeichers (40) eine vordefinierte Endposition (62) erreicht,
- eine innerhalb des Fluidbehälters (30) vorhandene elastische Membran (32) zur Druckbeaufschlagung des Fluids (25), wobei
- der Druckbehälter (35) in Fließverbindung mit der Kultivierkammer (10) steht.

2. Kultiviereinheit (1) nach Anspruch 1, **dadurch gekennzeichnet, dass** der nicht von dem Fluid (25) eingenommene Innenraum (33) des Fluidbehälters (30) jenseits der elastischen Membran (32) als Druckkammer zur Unterstützung der Verdrängung des Fluids (25) ausgebildet ist.

3. Kultiviereinheit (1) nach Anspruch 1 oder 2, **dadurch gekennzeichnet, dass** der nicht von dem Fluid (25) eingenommene Innenraum (33) des Fluidbehälters (30) jenseits der elastischen Membran (32) mit dem Druckbehälter (35) in Fließverbindung steht.

4. Kultiviereinheit (1) nach einem der vorangehenden Ansprüche, **dadurch gekennzeichnet, dass** zumindest eine Begrenzungsfläche einer Kultivierkammer (10) als flexible, gaspermeable Folie (51) ausgebildet ist.

5. Kultiviereinheit (1) nach einem der vorangehenden Ansprüche, **dadurch gekennzeichnet, dass** die Schmelzsicherung (60) aus niedrigschmelzenden Materialien aus Wachs, Kunststoff oder Metallen besteht, die eine Aktivierung bei Temperaturen unterhalb 70°C ermöglichen.

## Claims

1. Cultivation unit (1), comprising a cultivation chamber (10) as well as one or several supply units (20), which are in a flow connection therewith, for feeding in a fluid (25), **characterised in that** a supply unit (20) has the following characteristics:
- A fluid container (30) which is displaceable inside a pressure tank (35) under the influence of an elastic energy reservoir (40), with the fluid (25) that can be stored therein,
- a fusible cut-out (60) for activating the movement of the fluid container (30),
- a cannula (53) penetrating a septum (52) present at the drain of the fluid container (30) when the movement of the fluid container (30) under the influence of the elastic reservoir (40) reaches a predefined end position (62),
- an elastic membrane (32) present inside the fluid container (30) for pressurizing the fluid (25),
- the pressure tank (35) being in a flow connection with the cultivation chamber (10).

2. Cultivation unit (1) as claimed in claim 1, **characterised in that** the inner space (33) of the fluid container (30) that is not occupied by the fluid (25) is embodied, beyond the elastic membrane (32), as a pressure chamber to support the displacement of the fluid (25).

3. Cultivation unit (1) as claimed in claims 1 or 2, **characterised in that** the inner space (33) of the fluid container (30) that is not occupied by the fluid (25) is, beyond the elastic membrane (32), in a flow connection with the pressure tank (35).

4. Cultivation unit (1) as claimed in any one of the preceding claims, **characterised in that** at least one limitation surface of a cultivation chamber (10) is embodied as a flexible, gas-permeable film (51).

5. Cultivation unit (1) as claimed in any one of the preceding claims, **characterised in that** the fusible cut-out (60) consists of low-melting materials, of wax, plastics or metals, allowing an activation at temperatures below 70°C.

## Revendications

1. Unité de culture (1), comportant une chambre de culture (10) ainsi qu'une ou plusieures unités d'alimentation (20) pour amener un fluide (25), laquelle/lesquelles étant en connexion de flux avec celle, **caractérisée en ce qu'**une unité d'alimentation (20) comporte les caractéristiques suivantes :
- un récipient fluide (30), lequel est déplaçable dans un réservoir à pression (35) sous l'influence d'un réservoir d'énergie (40) élastique, avec le fluide (25), lequel peut être stocké là-dedans,
- un coupe-circuit fusible (60) pour activer le mouvement du récipient fluide (30),
- une canule (53) pénétrant un septum (52) présent au drain du récipient fluide (30) si le mouvement du récipient de fluide (30) arrive à une position finale (62) prédéfinie sous l'influence du réservoir d'énergie (40) élastique,
- une membrane élastique (32) présente dans le récipient fluide (30) pour pressuriser le fluide (25),
- le réservoir à pression (35) étant en connexion de flux avec la chambre de culture (10).

2. Unité de culture (1) selon la revendication 1, **caractérisée en ce que** l'espace intérieur (33) du récipient fluide (30) pas occupé par le fluide (25) est implémenté, au-delà de la membrane élastique (32), comme caisson hyperbare pour soutenir le déplacement du fluide (25).

3. Unité de culture (1) selon la revendication 1 ou 2, **caractérisée en ce que** l'espace intérieur (33) du récipient fluide (30) pas occupé par le fluide (25) est, au-delà de la membrane élastique (32), en connexion de flux avec le réservoir à pression (35).

4. Unité de culture (1) selon l'une quelconque des revendications précédentes, **caractérisée en ce qu'**au moins une surface limitant d'une chambre de culture (10) est implémentée comme un film (51) flexible, perméable à gaz.

5. Unité de culture (1) selon l'une quelconque des revendications précédentes, **caractérisée en ce que** le coupe-circuit fusible (60) est composé de matériaux de bas point de fusion, de cire, plastique ou métaux, lesquels matériaux permettent une activation à des températures au-dessous de 70°C.
